# EUROPEAN PATENT APPLICATION

(11) **EP 1 462 115 A1**
(43) Date of publication of application: **29.09.2004**
(21) Application number: 02791971.1
(22) Date of filing: 09.12.2002
(51) Int. Cl.: A61K 47/42, A61K 47/02, A61K 9/48, A23L 1/00

(54) **EDIBLE CAPSULES**

(30) Priority: 11.12.2001 JP 2001377163; 13.03.2002 JP 2002068569
(71) Applicant: Ajinomoto Co., Inc., Tokyo 104-8315 (JP)
(72) Inventor: FURUTA, Kiyonori, c/o AJINOMOTO CO., INC., Kawasaki-shi, Kanagawa 210-8681 (JP); MARUYAMA, Kentarou, c/o AJINOMOTO CO., INC., Kawasaki-shi, Kanagawa 210-8681 (JP); TAKAKURA, Yukiko, c/o AJINOMOTO CO., INC., Kawasaki-shi, Kanagawa 210-8681 (JP); SATO, Hiroyuki, c/o AJINOMOTO CO., INC., Kawasaki-shi, Kanagawa 210-8681 (JP)
(74) Representative: Nicholls, Kathryn Margaret
(86) International application number: PCT/JP2002/012879
(87) International publication number: WO 2003/049771

(57) **Abstract**

The problem of the present invention is to provide an edible capsule which is able to be used in the fields of pharmaceuticals, foods, health foods, etc., able to be ingested and composed of non-animal material. Another problem is to manufacture an acid-resistant capsule which comprises non-animal materials.

Means for solving the problem of the present invention is to prepare an edible capsule which is able to be ingested and composed of non-animal materials using polyglutamic acid and/or a salt thereof. It is also to prepare an acid-resistant capsule using polyglutamic acid and/or a salt thereof where weight-average molecular weight is more than 300,000.

## Description

### Technical Field

The present invention relates to an edible capsule which is utilized in the fields of pharmaceuticals, foods, health foods, etc. The edible capsule of the present invention is also applicable not only in the field of feeds for animals but also in the fields of cosmetics, toiletry and bath goods.

### Background Art

In edible capsules which are used in the fields of pharmaceuticals and foods, there are hard capsules which are suitable for enclosing powdery or granular pharmaceuticals, foods, etc. and soft capsules which are suitable for enclosing water-insoluble liquids such as oil liquid, pasty oil liquid and oil liquid in which powder is suspended. In addition, there have been known many microcapsules which are characterized in having a diameter of several µm to several hundred µm.

As a result of an increasing conscious to health in recent years, health foods such as a dietary supplement, a functional food has been receiving public attention. With regard to materials used for such health foods, there are various forms such as oily substance and powder. Capsule agent is used for the purpose of protection and stabilization of the content components contained in those health foods andmasking of taste and smell thereof. Hard capsules are mostly used for filling of powder, but filling of liquid substance therein is difficult. In the case of soft capsules, there are advantages that both liquid substance and powder are able to be filled and that various shapes are able to be selected by changing the mold tool. In view of the above, soft capsules are widely used in addition to hard capsules in the field of health foods. Moreover, microcapsules are widely used for the purpose of protection of unstable substances such as perfume, control of release of pharmaceuticals such as DDS, enclosure of substances having unusual taste and unusual flavor in food, etc.

On the other hand, in the conventional edible capsules, gelatin derived from animals such as cattle, swine, bird and fish has been used for a shell substrate thereof while there has been an increasing demand from the market for non-animal materials.

Under such circumstances, investigations have been carried out for the use of non-animal materials such as agar material, starch material and other various saccharides as materials for capsule shell but several problems such as that method for their manufacture is complicated, existing equipments are unable to be used, shell is hard and unable to be spread and shape of the capsule is limited have been pointed out and, therefore, they do not satisfy the characteristic required for capsules.

With regard to acid-resistant capsules, there have been known a method where surface of capsule made of gelatin derived from animals is coated with a substance which is insoluble in an acidic solution, a method where gelatin is mixed with a substance which is insoluble in an acidic solution, etc. but, since all of them use gelatin, there is a problem that they are unable to be said to be acid-resistant capsules comprising non-animal material.

On the other hand, with regard to a cross-linked product using a polyglutamic acid, there have been known a method of JP-A-10-251402 for a cross-linking method by means of radical polymerization by irradiation of radioactive ray, a method of JP-A-11-343339 for a cross-linking method using a polyepoxy compound, etc. but there are problems such as restriction in terms of equipments and legal restriction for utilization in the field of foods whereby there is a difficulty. In JP-A-2001-181387, a water-absorbing polymer where polyglutamic acid is cross-linked with metal is disclosed although there is no disclosure at all for its specific use, particularly as edible capsules.

### Disclosure of the Invention

The problem which is to be solved by the present invention is to manufacture an edible capsule which is able to be used in the fields of pharmaceuticals, foods, health foods, etc. and is composed of non-animal materials which can be ingested. Another problem to be solved is to manufacture an acid-resistant capsule which comprises non-animal materials.

The present inventors have found that the above-mentioned problems are able to be solved when polyglutamic acid and/or a salt thereof are/is used in edible capsules and achieved the present invention.

Thus, the present invention relates to an edible capsule containing polyglutamic acid and/or a salt thereof. The present invention also relates to an acid-resistant edible capsule which is characterized in that weight-average molecular weight of the polyglutamic acid and/or a salt thereof is more than 300,000.

### Brief Description of the Drawings

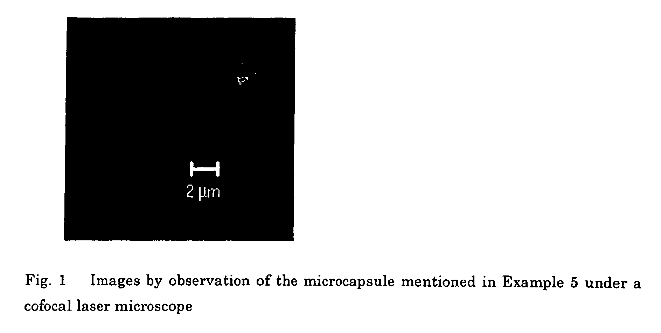

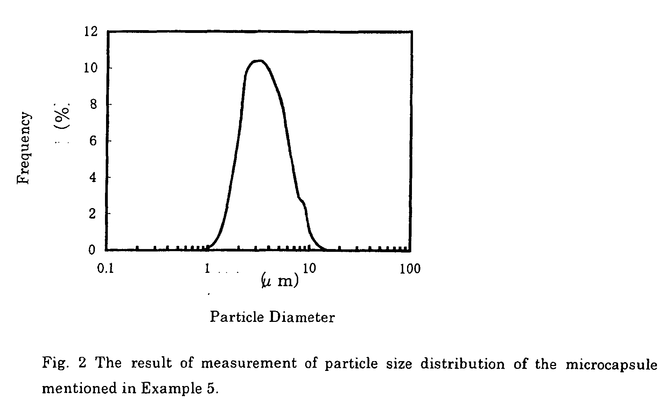

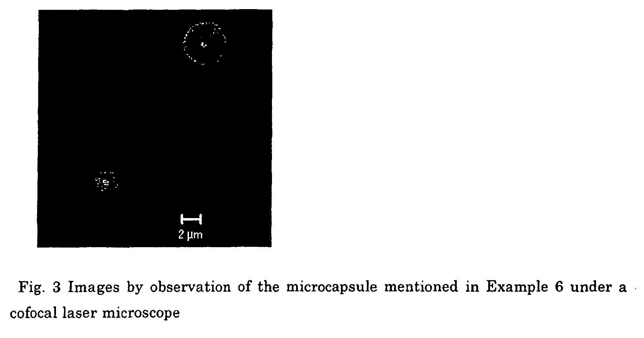

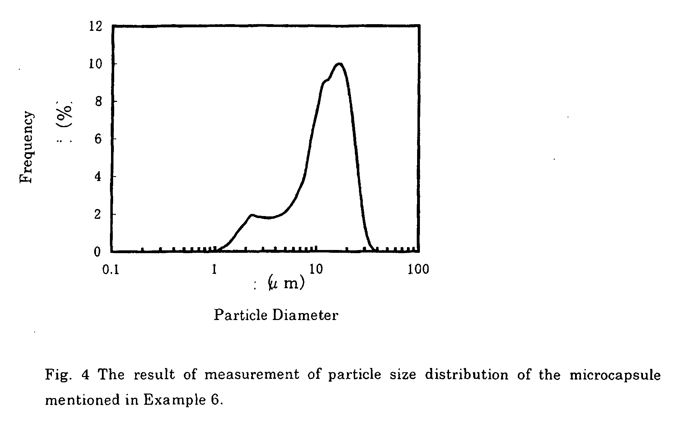

Fig. 1 is photographic picture of images by observation of the microcapsule mentioned in Example 5 under a confocal laser scanning microscope and Fig. 2 is a drawing which shows the result of measurement of particle size distribution of the microcapsule mentioned in Example 5. Fig. 3 is photographic picture of images by observation of the microcapsule mentioned in Example 6 under a confocal laser scanning microscope and Fig. 4 is a drawing which shows the result of measurement of particle size distribution of the microcapsule mentioned in Example 6.

### Best Mode for Carrying Out the Invention

With regard to polyglutamic acid and/or a salt thereof used in the present invention, anything may be used without particular limitation so far as it is a compound where glutamic acid is polymerized although poly-γ-glutamic acid and/or a salt thereof are/is preferred.

Polyglutamic acid is able to be manufactured by chemical synthesis and enzymatic synthesis and also by means of incubation using cells of genus *Bacillus* such as *Bacillus natto.* When *B. natto* or the like is used, polyglutamic acid contained in viscous substance of natto is extracted and used or polyglutamic acid which is extracellularly secreted from the cells is used.

Generally, polyglutamic acid in the viscous substance of natto or polyglutamic acid secreted by genus *Bacillus* under the conventional incubation condition is a high-molecular substance and is highly viscous and, therefore, it is able to be treated for making its molecular weight low by an acid or an enzyme depending upon the use.

For the purpose of releasing the content after being dissolved in stomach, it is preferred to prepare polyglutamic acid having a molecular weight of 10, 000 to 300,000 by making into low molecules using an acid or an enzyme.

For the purpose of releasing the content which is not resistant to an acid in small intestine or the like, it is necessary to give an acid-resistant property or, in other words, such a property that it is hardly soluble in stomach which is in an acidic condition having a low pH while easily soluble in digestive tract such as small intestine having a neutral pH. For such a use, it is preferred that the molecular weight of polyglutamic acid is more than 300,000.

Incidentally, the molecular weight used in the present invention is a weight-average molecular weight measured by a gel filtration - light scattering method (GPC-MALLS method; Dawn DPS manufactured by Wyatt Technology).

Polyglutamic acid used in the present invention may be free or a salt. Polyglutamate is able to be manufactured by the reaction of polyglutamic acid with a basic compound. At that time, there is no particular limitation for the basic compound but an alkaline metal or alkaline earth metal hydroxide such as sodium hydroxide, potassium hydroxide or magnesium hydroxide or an organic basic compound such as ammonia or amine may be used. With regard to the ratio in the reaction of the basic compound with polyglutamic acid, the reaction of the basic compound within a range of 0.1 to 1.0 equivalent may be carried out to one equivalent of carboxylic acid in polyglutamic acid.

With regard to the metal used for the formation of a cross-linked product of polyglutamic acid, a divalent or higher metal which is able to react with plural carboxyl groups in polyglutamic acid is used. Preferably, calcium, iron, aluminum, chromium, etc. may be used.

It is also possible to use a metal compound containing such a metal. Although there is no particular limitation for the metal compound, it is preferred to use a calcium compound, an aluminum compound and/or a double salt thereof. To be more specific, calcium chloride, calcium hydroxide, calcium carbonate, aluminum potassium sulfate, aluminum potassium sulfate dodecahydrate (potassium alum), aluminum ammonium sulfate, aluminum ammonium sulfate dodecahydrate (ammonium alum), etc. may be exemplified.

With regard to polyglutamic acid or the like and the above metal compound, their mixing may be carried out in a rate of 100 parts by weight of polyglutamic acid and 1 mmol to 100 mmol of the metal compound or, preferably, 5 mmol to 75 mmol. Such a mixing rate varies depending upon the molecular weight of polyglutamic acid and the species of the metal whereby it may be appropriately decided.

The polyglutamic acid shell which constitutes the edible capsules of the present invention may be constituted by addition of polyglutamic acid or a cross-linked product of polyglutamic acid, a plasticizer and water. Examples of the plasticizer are sorbitol, mannitol, glycerol and 1, 3-butylene glycol. Although addition of the plasticizer is not essential, it is added usually within a range of from 5 to 60 parts or, preferably, from 10 to 50 parts by weight to 100 parts by weight of polyglutamic acid. Besides the above, a disintegration promoter, a stabilizer, a coloring agent, perfume, etc. may be added to the polyglutamic acid shell if necessary. In addition, shell materials for capsules which have been known and used already may be also used together therewith.

The edible capsule of the present invention is appropriate to any of various capsules such as hard capsules, soft capsules and seamless soft capsules.

With regard to a method for the manufacture of hard capsules, a dipping method where a mold tool (mold pin) is dipped in a solution containing shell substrate followed by drying, etc. may be used. With regard to a method for the manufacture of soft capsules, a rotary die method which is a kind of a stamping method may be used. That is a method where two sheets are used and molding of the capsule, filling of the content and heat sealing are carried out at the same time. With regard to a method for the manufacture of seamless soft capsules, a hardening-in-the-liquid method which is a kind of a dripping method may be used. That is a method where an content liquid for capsule and a solution containing shell substrate flow out at a predetermined rate from inner and outer nozzles, respectively, in the double or more nozzles, the liquid flow in two layers as such is cut with a predetermined interval to give liquid droplets and then a shell layer for the outside is gelled to make into a capsule. With regard to a method for the manufacture of microcapsules, a coacervation method (a phase separation method), an emulsion method (a stirring emulsifying method, an ultrasonic emulsifying method), a spray-drying method, etc. may be used.

With regard to shape, size, etc. of the edible capsule, there is no particular limitation and, as to the shape, it is possible to manufacture in a round shape, an oval shape, an oblong shape, a tube shape, a teardrop shape, etc. As to the size, it is possible to manufacture the capsule in a size from about several µm to about several cm.

As mentioned above, the manufacture may be carried out using a conventionally known manufacturing method depending upon the intention for use, the necessary shape and size of the edible capsule.

Incidentally, according to Japanese Patent Laid-Open No. 03/030,648, Japanese Patent Laid-Open No. 05/316,999, Japanese Patent No. 3,232,718, U. S. Patent No. 5,447,732 and European Patent No. 605,757, it has been known that poly-γ-glutamic acid contained in a viscous substance of natto has an effect of promoting the absorption of minerals such as calcium and iron into the body.

Accordingly, the edible capsule of the present invention containing polyglutamic acid and/or a salt thereof is expected to have a function of promoting the absorption of mineral components filled therein as a content or of mineral components ingested as foods in addition to the function which has been already available in the conventional capsules.

<Examples> The present invention will now be illustrated in more detail by way of the following Manufacturing Examples and Examples although the present invention is not limited to those Manufacturing Examples and Examples.

### <Manufacturing Example 1> Manufacture of poly-γ-glutamic acid (molecular weight: 30,000)

*Bacillus natto* (Accession Number at the National Institute of Bioscience and Human Technology, Agency of Industrial Science and Technology: FERM P-10607) belonging to *Bacillus subtilis* was subjected to a seed culture at 32°C for 24 hours in a culture solution (pH 6.0) containing 0.3% of malt extract, 0.3% of yeast extract, 0.5% of polypeptone and 1.0% of glucose using a three-liter mini-jar. After that, the seed-cultured solution was inoculated in an amount of 0.5% to a culture solution (pH 6.4) containing 7.5% of glucose, 1.5% of ammonium sulfate, 0.15% of magnesium sulfate, 5.0% of monosodium glutamate and 1.0% of sodium chloride using a 500-liter jar and incubated at 37°C for 48 hours where number of revolutions was 250 rpm and aeration was 0.5 to 1 vvm. The culture solution after the incubation was subjected to a GPC method to measure the amount of poly-γ-glutamic acid and it was found to be 3.0 g/dl. The resulting culture solution (50 liters) was adjusted to pH 2 using a concentrated hydrochloric acid, filtered through a precise filtration membrane (PSP-313 manufactured by Asahi Chemical Industry; pore size: 0.1 µm; membrane area: 6.0 m²) and heated at 50°C for 6 hours to make the molecular weight low. After cooling, it was neutralized with sodium hydroxide and desalted using an ultrafilter membrane (SIP-3013 manufactured by Asahi Chemical Industry; fractionating molecular weight: 6,000; membrane area: 4.7 m²) together with addition of water in the same amount as the permeated solution to the circulating solution. When the amount of the adding water became 50 liters, desalting was finished and concentration was carried out until the amount of e circulating solution became 8 liters. This was further concentrated *in vacuo* to give an aqueous solution of poly-γ-glutamic acid. Its concentration by a GPC method was 16% and weight-average molecular weight by a GPC-MALLS method was 30, 000. The resulting aqueous solution of poly-γ-glutamic acid was dried using a vacuum drum drier (drum heating temperature: 120°C; degree of valuation: 10 Torr; drying time: 30 seconds) to give poly-γ-glutamic acid.

### <Manufacturing Example 2> Manufacture of poly-γ-glutamic acid (molecular weights: 260, 000, 490, 000 and 800,000)

After the culture solution was prepared according to the same method as in Manufacturing Example 1, it was subjected to a pH adjustment and a precise filtration. The same operation as in Manufacturing Example 1 was carried out except that the treatment for making the molecular weight low was conducted at 50°C for 0, 10 or 30 minute (s) to give poly-γ-glutamic acid. Weight-average molecular weights by a GPC-MALLS method were 800,000, 490,000 and 260,000, respectively.

Characteristic values shown in Examples and Comparative Examples were measured and judged according to the following methods.
<Appearance> It was judged by naked eye.
<Appropriateness for molding> It was judged by naked eye whether molding of capsules was possible.
<Viscosity of the solution containing shell substrate> It was measured at 25°C using a viscometer of type B (manufactured by K. K. Tokyo Keiki).
<Water content of the shell> It was measured under the condition of 105°C for 2 hours using an infrared device for measuring the water content (Kett FD-600).
<Solubility test> A capsule was poured into water which was warmed at 40 ± 1°C and allowed to stand, the dissolving state of the capsule was observed and time until it was completely dissolved was measured.
<Disintegration test> In accordance with the disintegration test method mentioned in the Japanese Pharmacopoeia, a disintegration test was carried out according to the following methods using the first solution (artificial gastric juice) and the second solution (artificial intestinal juice).

### 1. Test solution

First solution: Sodium chloride (2 g), 7 ml of hydrochloric acid and water were mixed and dissolved to make one liter (pH 1.2).

Second solution: A 0.2 mol/liter sodium hydroxide reagent (118 ml) and water were added to 250 ml of a 0.2 mol/liter potassium dihydrogen phosphate reagent to make one liter (pH 6.8).

### 2. Test solution temperature and device:

Temperature of the test solution: 37 ± 1°C
Disintegration test device: NT-4HSF manufactured by Toyama Sangyo

### 3. Criteria for the judgment

Opening time: time until the content came out from the connected part
Disintegration time: time until all of the shell was dissolved
oo = completely dissolved within five minutes
o = completely dissolved within ten minutes
× = 10 minutes or more were need for complete dissolving

### <Example 1>

A solution containing a cross-linked substance of poly-γ-glutamic acid as shell substrate was prepared according to the following composition. Poly-γ-glutamic acid, deionized water and glycerol were added using a one-liter beaker followed by stirring. Then a 6N hydrochloric acid was added with stirring to prepare a poly-γ-glutamic acid solution. Further, 0.2M aluminum potassium sulfate dodecahydrate was added thereto followed by stirring and then a 10N sodium hydroxide was added thereto followed by stirring. When sodium hydroxide was added followed by stirring, viscosity of the solution simultaneously increased. When no more change in the viscosity was noted, stirring was finished to give a solution containing a cross-linked substance of poly-γ-glutamic acid as shell substrate.

| | |
|---|---|
| Poly-γ-glutamic acid of Manufacturing Example 1 | 160 parts |
| Deionized water | 200 parts |
| Glycerol (special reagent grade; manufactured by Wako Pure Chemicals) | 40 parts |
| 6N Hydrochloric acid (special reagent grade; manufactured by Wako Pure Chemicals) | 48 parts |
| 0.2M Aluminum potassium sulfate dodecahydrate | 125 parts |
| 10N Sodium hydroxide | 21 parts |

Viscosity of the above solution containing shell substrate was 12,400 cps. The solution containing shell substrate was defoamed *in vacuo,* coated on a glass plate using an applicator of 2 mm thickness and air-dried for 24 hours in a chamber kept at constant temperature and humidity of 25°C and 45% RH. Thickness and water content of the resulting sheet were 0.7 mm and 23%, respectively.

The sheet was made into edible capsules according to a conventional method using a rotary capsule molding machine (manufactured by Kamata Co., Ltd.). With regard to a metal mold, No. 5 of an oval type was used while, with regard to a filler, soybean oil was used. The result was that the sheet containing a cross-linked poly-γ-glutamic acid substance was able to be made into good capsules. It was also made clear that the edible capsules using a cross-linked poly-γ-glutamic acid substance had the same appearance and molding adaptability as those of the conventional edible capsules using gelatin.

After that, the resulting edible capsules were allowed to stand in water of 40°C and a dissolving test was carried out whereupon all shells were dissolved within 16 minutes and the contents were discharged.

### <Example 2>

A solution containing a cross-linked substance of poly-γ-glutamic acid as shell substrate was prepared according to the following composition. Poly-γ-glutamic acid, deionized water and glycerol were added using a one-liter beaker followed by stirring. Then a 6N hydrochloric acid was added with stirring to prepare a poly-γ-glutamic acid solution. Further, 0.2M aluminum potassium sulfate dodecahydrate was added thereto followed by stirring and then a 10N sodium hydroxide was added thereto followed by stirring. When sodium hydroxide was added followed by stirring, viscosity of the solution simultaneously increased. When no increase in the viscosity was noted, stirring was finished to give a solution containing a cross-linked substance of poly-γ-glutamic acid as shell substrate.

| | |
|---|---|
| Poly-γ-glutamic acid of Manufacturing Example 1 | 170 parts |
| Deionized water | 102 parts |
| Glycerol (special reagent grade; manufactured by Wako Pure Chemicals) | 43 parts |
| 6N Hydrochloric acid (special reagent grade; manufactured by Wako Pure Chemicals) | 85 parts |
| 0.2M Aluminum potassium sulfate dodecahydrate | 170 parts |
| 10N Sodium hydroxide | 42.5 parts |

The solution containing shell substrate was warmed at 60°C and defoamed *in vacuo,* coated on a flat plate to which a mold-releasing tape was adhered using an applicator of 3 mm thickness and air-dried for 24 hours in a chamber kept at constant temperature and humidity of 22°C and 25% RH. Thickness and water content of the resulting sheet were 0.7 mm and 23%, respectively. The same operation as in Example 1 was carried out except that a medium-chain fatty acid triglyceride was used as a filler whereupon edible capsules were prepared.

The resulting sheet were able to be made into good capsules. It was also made clear that the edible capsules using a cross-linked poly-γ-glutamic acid substance had the same appearance and molding adaptability as those of the conventional edible capsules using gelatin.

After that, the resulting edible capsules were allowed to stand in water of 40°C and a dissolving test was carried out whereupon all shells were dissolved within 16 minutes and the contents were discharged.

Results of the disintegration test for the edible capsules of Example 2 (mean values of six samples) are shown in Table 1.

**(Table 1)**

| Test Solutions | | Example 2 | Comparative Example |
|---|---|---|---|
| Artificial Gastric Juice | Opening Time | 3 minutes and 37 seconds | 3 minutes and 19 seconds |
| | Disintegration Time | 0 | 0 |
| Artificial Intestinal Juice | Opening Time | 2 minutes and 37 seconds | 4 minutes and 22 seconds |
| | Disintegration Time | 00 | × |

### <Comparative Example>

A solution containing shell substrate was prepared according to the following composition. Deionized water was added to gelatin to swell at room temperature for 1 hour, then glycerol was added thereto and the mixture was heated at 60°C on a water bath followed by stirring whereupon a gelatin solution was prepared.

| | |
|---|---|
| Gelatin (derived from animal; jelly strength: 150 blooms) | 100 parts |
| Glycerol | 35 parts |
| Deionized water | 100 parts |

The solution containing shell substrate prepared according to the above composition was defoamed *in vacuo* at 60°C. Thickness of the resulting sheet was 0.85 mm. The same operation as in Examples 1 was carried out to give edible capsules constituted from materials derived from animal.

The resulting edible capsules were subjected to a dissolving test by being allowed to stand in water of 40°C. The result was that, even after 30 minutes, the shell and the content remained. Results of the disintegration test of the edible capsules of this Comparative Example (mean values of six samples) are shown in Table 1.

### <Example 3>

An example for the manufacture of seamless soft capsules by a hardening-in-liquid method will be shown as hereunder.

To 100 parts by weight of poly-γ-glutamic acid of Manufacturing Example 1 were added 80 parts of deionized water and the pH was adjusted to 4.9 with a 10N aqueous solution of sodium hydroxide to prepare a solution of poly-γ-glutamic acid. To 100 parts of the resulting solution of PGA were added 100 ml of a 2M aqueous solution of calcium chloride to prepare a solution containing poly-γ-glutamic acid as shell substrate. Soybean oil was used as a content liquid while ethanol was used as a hardening solution and a solution containing poly-γ-glutamic acid as shell substrate and the content liquid were dropped into a hardening solution from a double nozzle (diameter of the outer nozzle was 3 mm while that of the inner nozzle was 2 mm). The dropping rate at that time was 4.7 ml/minute for the solution containing poly-γ-glutamic acid as shell substrate and was 3.9 ml/minute for the soybean oil. Spherical capsules were formed. The capsules were taken out from the hardening solution and dried for 12 hours at room temperature to give seamless soft capsules. The resulting seamless soft capsules were allowed to stand in water of 40°C to carry out a dissolving test. The result was that all shells were dissolved within 3.5 minutes and the content was discharged.

### <Example 4>

An example for the manufacture of hard capsules using a dipping method will be shown as hereunder.

To 100 parts of poly-γ-glutamic acid (molecular weight: 800,000) obtained in Manufacturing Example 2 were added 700 parts of deionized water and swelling was conducted at room temperature for 30 minutes followed by stirring to give a solution containing poly-γ-glutamic acid as shell substrate. The solution containing shell substrate was allowed to stand at room temperature for 2 hours to defoam. A dipping pin made of stainless steel where the end was rounded was dipped thereinto vertically, taken out and dried with hot air of 80°C by rotating for making the thickness of the solution containing shell substrate uniform until fluidity was no longer noted. After that, it was dried for 12 hours at 25°C and 45% relatively humidity. The capsule shell obtained after drying was taken out from the dipping pin and cut into a desired length to give an empty capsule. Thickness of the capsule shell was 0.15 mm. The resulting empty capsule was soft and deformable and did not generate crack or the like.

<Acid-resistant test> will be shown as hereunder.
A. To 100 parts of poly-γ-glutamic acid (molecular weight: 260,000) prepared in Manufacturing Example 2 were added 600 parts of deionized water followed by stirring to give a solution containing poly-γ-glutamic acid as shell substrate (A).
B. To 100 parts of poly-γ-glutamic acid (molecular weight: 490, 000) prepared in Manufacturing Example 2 were added 600 parts of deionized water and then swelling was conducted at room temperature for 30 minutes followed by stirring to give a solution containing poly-γ-glutamic acid as shell substrate (B).
C. To 100 parts of poly-γ-glutamic acid (molecular weight: 800,000) prepared in Example 4 were added 700 parts of deionized water and then swelling was conducted at room temperature for 30 minutes followed by stirring to give a solution containing poly-γ-glutamic acid as shell substrate(C).

Each of the above solutions (A), (B) and (C) was coated on a glass plate so as to make the membrane thickness after drying 0.15 mm and dried for 12 hours at 25°C and 45% relative humidity. The sheet was peeled off from the glass plate and cut into a size of 2 mm square to give test pieces.

The test piece (50 mg) was placed in each 50 ml of the first solution (artificial gastric juice) and the second solution (artificial intestinal juice) stipulated by the Japanese Pharmacopoeia and allowed to stand at 37°C and the time until it was dissolved was measured. The result is shown in Table 2.

**(Table 2)**

| Test Solutions | A (M. W. = 260,000) | B (M. W. = 490,000) | C (M. W. = 800,000) |
|---|---|---|---|
| Artificial Gastric Solution | × | 0 | 0 |
| Artificial Intestinal Solution | × | × | × |
| Criteria for judging the acid-resistant solubility: | | | |
| × = dissolved within 10 minutes | | | |
| o = insoluble during 30 minutes or longer | | | |

### <Example 5>

Microcapsules containing a cross-linked poly-γ-glutamic acid substance were prepared according to the following composition. Firstly, deionized water was added to poly-γ-glutamic acid (molecular weight: 26,000) which was separately manufactured by a method of Manufacturing Example 1 so as to make its concentration 10% (w/w) followed by stirring. After that, 27 ml of 10% (w/w) poly-γ-glutamic acid solution and 3 ml of soybean oil were added to a 50-ml stainless steel tube and subjected to an emulsifying dispersion for 1 minute at output of 160 watts using an ultrasonic treating machine (Branson Sonifier 250). After 1 minute, 2 ml of a 0.2M aluminum potassium sulfate dodecahydrate solution were dropped thereinto without stopping the ultrasonic treatment and then an ultrasonic wave treatment was carried out for 2 minutes to give a milky white dispersion.

| | |
|---|---|
| 10% (w/w) Poly-γ-glutamic acid solution | 27 parts |
| Soybean oil | 3 parts |
| 0.2M Aluminum potassium sulfate dodecahydrate | 2 parts |

When the above dispersion where the soybean oil was subjected to a fluorescent dyeing with Nile Red while the poly-γ-glutamic acid was subj ected to a fluorescent dyeing with Rhodamine was observed under a confocal laser scanning microscope whereupon the presence of microcapsules enclosing soybean oil therein was confirmed (Fig. 1). When particle size distribution of the capsule dispersion was measured using a particle size distribution meter of a laser diffraction type (LA 920 of Horiba), a median diameter was so small as 3.2 µm and was a single peak (Fig. 2).

### <Example 6>

Microcapsules containing a cross-linked poly-γ-glutamic acid substance were prepared according to the following composition. Firstly, deionized water was added to poly-γ-glutamic acid (molecular weight: 26,000) separately manufactured by a method of Manufacturing Example 1 so as to make its concentration 10% (w/w) followed by stirring. After that, 27 ml of 10% (w/w) poly-γ-glutamic acid solution and 3 ml of soybean oil were added to a 50-ml stainless steel tube and subjected to an emulsifying dispersion for 1 minute at 15,000 rpm using a homogenizer (Kinematica PT 3000). After 1 minute, 2 ml of a 0.2M aluminum potassium sulfate dodecahydrate solution were dropped thereinto without stopping the ultrasonic treatment and then an ultrasonic wave treatment was carried out for 2 minutes to give a milky white dispersion.

| | |
|---|---|
| 10% (w/w) Poly-γ-glutamic acid solution | 27 parts |
| Soybean oil | 3 parts |
| 0.2M Aluminum potassium sulfate dodecahydrate | 2 parts |

When the above dispersion where the soybean oil was subjected to a fluorescent dyeing with Nile Red while the poly-γ-glutamic acid was subjected to a fluorescent dyeing with Rhodamine was observed under a confocal laser scanning microscope whereupon the presence of microcapsules enclosing soybean therein was confirmed (Fig. 3). When particle size distribution of the capsule dispersion was measured using a particle size distribution meter of a laser diffraction type (LA 920 of Horiba), a median diameter was 11.7 µm (Fig. 4).

### Industrial Applicability

When polyglutamic acid and/or a salt thereof are/is used, it is now possible to give edible capsules being constituted from non-animal materials. The resulting edible capsules have the same appropriateness of molding as conventional capsules and have excellent disintegrating property and solubility and an acid resistance is also able to be given thereto when molecular weight is duly selected.

## Claims

1. An edible capsule containing polyglutamic acid and/or a salt thereof.

2. An edible capsule containing polyglutamic acid and/or a salt thereof in its shell.

3. The edible capsule according to claim 1 or 2, wherein the polyglutamic acid and/or a salt thereof are/is polyglutamic acid substance(s) being cross-linked with metal.

4. The edible capsule according to claim 3, wherein the metal is a metal salt or double salt selected from calcium, iron, aluminum and chromium.

5. The edible capsule according to claims 1 to 4, wherein the polyglutamic acid and/or a salt thereof are/is poly-γ-glutamic acid and/or a salt thereof.

6. The edible capsule according to claims 1 to 5, wherein weight-average molecular weight of the polyglutamic acid and/or a salt thereof is from 10,000 to 300,000.

7. The edible capsule according to claims 1 to 5, wherein weight-average molecular weight of the polyglutamic acid and/or a salt thereof is more than 300,000.
